# EUROPEAN PATENT SPECIFICATION

(11) **EP 3 157 437 B1**
(45) Date of publication and mention of the grant of the patent: **15.08.2018**
(21) Application number: 15810485.1
(22) Date of filing: 15.06.2015
(51) Int. Cl.: A61B 8/00, A61B 8/12, A61B 8/08, A61B 8/14, A61B 5/00

(54) **DESIGN AND METHOD FOR INTRAVASCULAR CATHETER**
SYSTEM UND VERFAHREN FÜR INTRAVASKULÄREN KATHETER
CONCEPTION ET PROCÉDÉ POUR CATHÉTER INTRAVASCULAIRE

(30) Priority: 17.06.2014 US 201462013448 P
(43) Date of publication of application: 26.04.2017
(73) Proprietor: Koninklijke Philips N.V., 5656 AE Eindhoven (NL)
(72) Inventor: CURRLIN, Arnoldo, Murrieta, California 92563 (US); BURKETT, David H., Temecula, California 92591 (US)
(74) Representative: Steffen, Thomas
(86) International application number: PCT/US2015/035744
(87) International publication number: WO 2015/195505

(56) References cited:
- US-A- 4 686 982
- US-A- 4 794 931
- US-A- 5 842 993
- US-A1- 2008 161 696
- US-A1- 2008 177 139
- US-A1- 2009 264 768
- US-A1- 2013 218 020
- US-A1- 2013 218 020
- US-A1- 2013 303 919
- US-A1- 2014 107 488
- US-A1- 2014 107 489

## Description

### TECHNICAL FIELD

The present disclosure relates generally to elongate catheters for a rotational probe for insertion into a vessel, and in particular, to an intravascular ultrasound (IVUS) imaging catheter.

### BACKGROUND

Intravascular ultrasound (IVUS) imaging is widely used in interventional cardiology as a diagnostic tool for a diseased vessel, such as an artery, within the human body to determine the need for treatment, to guide the intervention, and/or to assess its effectiveness. IVUS imaging uses ultrasound echoes to create an image of the vessel of interest. The ultrasound waves pass easily through most tissues and blood, but they are partially reflected from discontinuities arising from tissue structures (such as the various layers of the vessel wall), red blood cells, and other features of interest. The IVUS imaging system, which is connected to an IVUS catheter by way of a patient interface module (PIM), processes the received ultrasound echoes to produce a cross-sectional image of the vessel where the catheter is placed.

In a typical rotational IVUS catheter, a single ultrasound transducer element fabricated from a piezoelectric ceramic material is located at the tip of a flexible drive shaft that spins inside a plastic sheath inserted into the vessel of interest. The transducer element is oriented such that the ultrasound beam propagates generally perpendicular to the axis of the catheter. The fluid-filled sheath protects the vessel tissue from the spinning transducer and drive shaft while permitting ultrasound signals to freely propagate from the transducer into the tissue and back. As the drive shaft rotates (typically at 30 revolutions per second), the transducer is periodically excited with a high voltage pulse to emit a short burst of ultrasound. The same transducer then listens for the returning echoes reflected from various tissue structures, and the IVUS imaging system assembles a two dimensional display of the vessel cross-section from a sequence of several hundred of these ultrasound pulse/echo acquisition sequences occurring during a single revolution of the transducer.

A typical drive shaft is made with stainless steel wires with a hollow core where electrical cables are placed inside the hollow core to electrically couple the transducer to the IVUS imaging system at the patience interface module (PIM). As the drive shaft can be made quite long for certain applications, e.g., in the range of 100 centimeter (cm) to 250 cm, threading the electrical cables through the hollow core can be a difficult task. Furthermore, due to size limitations, the drive shaft has to be unfinished at both ends, requiring that the termination or final connections of the electrical cables in the IVUS catheter be made by hand after threading the electrical cables through the drive shaft. Such tasks are difficult and time consuming.

Accordingly, there remains a need for improved devices, systems, and methods for providing a compact and efficient drive shaft in an intravascular ultrasound system.

### SUMMARY

Embodiments of the present disclosure provide a compact and efficient drive shaft in an intravascular ultrasound system.

In an embodiment, an elongate catheter for a rotational probe for insertion into a vessel is provided. The elongate catheter comprises a flexible body; a proximal connector adjacent a proximal portion of the flexible body; and an elongate shaft disposed within the flexible body, the shaft having a drive cable and a work element coupled to the drive cable adjacent a distal portion of the flexible body, the drive cable having a torque transmission core and at least one conductor disposed lengthwise outside of the torque transmission core, and the at least one conductor coupling the work element to a proximal portion of the elongate shaft. In some instances, the at least one conductor is an electrical conductor. In some instances, the at least one conductor is an optical fiber. The number of conductors depends on the application. For example, there may be two conductors or four conductors in the drive cable in some applications.

In some instances, the drive cable further comprises an electrical insulating layer between the at least one conductor and the torque transmission core. In some instances, the drive cable further comprises a polymer jacket, the polymer jacket securing the at least one conductor to the torque transmission core. In some instances, the drive cable further comprises a plurality of polymer bands, the plurality of polymer bands securing the at least one conductor to the torque transmission core. In some embodiments, the at least one conductor is embedded in a polymer jacket that is secured to the torque transmission core.

In some embodiments, the torque transmission core of the drive cable is made with stainless steel. In some embodiments, the torque transmission core of the drive cable is an optical fiber and the at least one conductor is an electrical conductor. In some embodiments, the work element of the elongate catheter is a piezoelectric micro-machined ultrasound transducer (PMUT) or a capacitive micro-machined ultrasound transducer (CMUT).

In another embodiment, a rotational probe for insertion into a vessel is provided. The probe includes an elongate catheter having a flexible body, a proximal connector adjacent a proximal portion of the flexible body, and an elongate shaft disposed within the flexible body, the shaft having a drive cable and a work element coupled to the drive cable adjacent a distal portion of the flexible body, the drive cable having a torque transmission core and at least one conductor disposed lengthwise outside of the torque transmission core, and the at least one conductor coupling the work element to a proximal portion of the elongate shaft; and an interface module configured to interface with the proximal connector of the elongate catheter, the interface module including: a spinning element configured to be fixedly coupled to a proximal portion of the shaft; a stationary element positioned adjacent to and spaced from the spinning element, wherein the stationary element is configured to pass signals to and receive signals from the work element through the spinning element; and a motor coupled to the spinning element for rotating the spinning element and the shaft when the spinning element is fixedly coupled to the proximal portion of the shaft.

In another embodiment, a method of manufacturing a catheter for a rotational probe for insertion into a vessel is provided. The method includes: providing an elongate torque transmission core; and securing at least one conductor to the elongate torque transmission core lengthwise. In some instances, the method further includes, before securing the at least one conductor to the elongate torque transmission core, forming an electrical insulating layer over the elongate torque transmission core, wherein the at least one conductor is placed adjacent to the electrical insulating layer. In some instances, the method further includes securing a polymer jacket over both the at least one conductor and the elongate torque transmission core. In some instances, the method further includes securing a plurality of polymer bands over both the at least one conductor and the elongate torque transmission core.

In some embodiments, the at least one conductor is embedded in a polymer jacket and the securing the at least one conductor includes securing the polymer jacket over the elongate torque transmission core. In that regard, securing the polymer jacket includes heat shrinking the polymer jacket over the elongate torque transmission core, or extruding the polymer jacket over the elongate torque transmission core. In some embodiments, the securing the at least one conductor includes co-extruding a polymer jacket and the at least one conductor over the elongate torque transmission core.

In some instances, the method further includes coupling a distal portion of the at least one conductor to a work element; and securing a distal portion of the torque transmission core to a housing that holds the work element. In that regard, the work element is a transducer in some embodiments.

Some embodiments of the present disclosure provide a compact and efficient drive cable in an intravascular ultrasound (IVUS) system. The drive cable is flexible yet with requisite torque for insertion into a vessel of interest. With conductors disposed outside a torque transmission core, the drive cable is easier to manufacture than the existing drive cables where electrical wires need to be threaded therein. In some embodiments, the conductors of the provided drive cable can be terminated in a subassembly in an early step of the manufacturing process, simplifying the tasks of making and/or using the drive cable downstream. Furthermore, since there is no need to thread wires through the torque transmission core, the dimensions and tolerance of the drive cable can be reduced, allowing for more space for additional components for the IVUS system. In addition or alternatively, the drive cable can be made stronger, allowing for more reliable operation and longer usable life.

In another embodiment, an elongate catheter for a rotational probe for insertion into a vessel is provided. The elongate catheter includes a flexible body; a proximal connector adjacent a proximal portion of the flexible body; and an elongate shaft disposed within the flexible body. The elongate shaft includes a drive cable and a work element coupled to the drive cable adjacent a distal portion of the flexible body. The drive cable includes a dielectric insulating layer, at least two conductors disposed lengthwise inside the dielectric insulating layer, a shield over the dielectric insulating layer, and an outer sheath over the shield. The at least two conductors couple the work element to a proximal portion of the elongate shaft. In some instances, the drive cable includes four conductors. In some instances, the drive cable further includes a strengthening layer embedded in the dielectric insulating layer and the strengthening layer can be made an electrical shield for the at least two conductors. In various instances, the drive cable of this embodiment provides a one-piece design for both data signal transmission and torque transmission, eliminating the need for a separate torque transmission core. Additional aspects, features, and advantages of the present disclosure will become apparent from the following detailed description.

### BRIEF DESCRIPTION OF THE DRAWINGS

Illustrative embodiments of the present disclosure will be described with reference to the accompanying drawings, of which:
FIG. 1 is a simplified fragmentary diagrammatic view of a rotational IVUS probe, according to some embodiments.
FIG. 2 is a simplified fragmentary diagrammatic view of an embodiment of an interface module and catheter for the rotational IVUS probe of FIG. 1, in accordance with an embodiment.
FIG. 3A is a diagrammatic, cross-sectional side view of a distal portion of the rotational IVUS probe of Fig. 1, in accordance with an embodiment.
FIG. 3B is a diagrammatic top view of a work element coupled to a distal portion of a drive cable, in accordance with an embodiment.
FIG. 4A is a diagrammatic perspective view of a drive cable, according to various aspects of the present disclosure.
FIG. 4B is a diagrammatic cross-sectional view of a drive cable, according to various aspects of the present disclosure.
FIG. 4C is a diagrammatic cross-sectional view of a drive cable, according to various aspects of the present disclosure.
FIG. 4D is a diagrammatic schematic view of a drive cable, according to various aspects of the present disclosure.
FIG. 5 is a method of manufacturing a catheter, according to various aspects of the present disclosure.
FIG. 6 is a diagrammatic, cross-sectional side view of a distal portion of the rotational IVUS probe of Fig. 1, in accordance with an embodiment.
FIG. 7 is a diagrammatic cross-sectional view of an embodiment of the drive cable in FIG. 6, according to various aspects of the present disclosure.
FIG. 8 is a diagrammatic cross-sectional view of another embodiment of the drive cable in FIG. 6, according to various aspects of the present disclosure.

### DETAILED DESCRIPTION

For the purposes of promoting an understanding of the principles of the present disclosure, reference will now be made to the embodiments illustrated in the drawings, and specific language will be used to describe the same. It is nevertheless understood that no limitation to the scope of the disclosure is intended. Any alterations and further modifications to the described devices, systems, and methods, and any further application of the principles of the present disclosure are fully contemplated and included within the present disclosure as would normally occur to one skilled in the art to which the disclosure relates. In particular, it is fully contemplated that the features, components, and/or steps described with respect to one embodiment may be combined with the features, components, and/or steps described with respect to other embodiments of the present disclosure. For the sake of brevity, however, the numerous iterations of these combinations will not be described separately.

As used herein, "flexible elongate member" or "elongate flexible member" includes at least any thin, long, flexible structure that can be inserted into the vasculature of a patient. While the illustrated embodiments of the "flexible elongate members" of the present disclosure have a cylindrical profile with a circular cross-sectional profile that defines an outer diameter of the flexible elongate member, in other instances all or a portion of the flexible elongate members may have other geometric cross-sectional profiles (*e.g.,* oval, rectangular, square, elliptical, etc.) or non-geometric cross-sectional profiles. Flexible elongate members include, for example, guidewires and catheters. In that regard, catheters may or may not include a lumen extending along its length for receiving and/or guiding other instruments. If the catheter includes a lumen, the lumen may be centered or offset with respect to the cross-sectional profile of the device.

In most embodiments, the flexible elongate members of the present disclosure include one or more electronic, optical, or electro-optical components. For example, without limitation, a flexible elongate member may include one or more of the following types of components: a pressure sensor, a temperature sensor, an imaging element, an optical fiber, an ultrasound transducer, a reflector, a mirror, a prism, an ablation element, an RF electrode, a conductor, and/or combinations thereof. Generally, these components are configured to obtain data related to a vessel or other portion of the anatomy in which the flexible elongate member is disposed. Often the components are also configured to communicate the data to an external device for processing and/or display. In some aspects, embodiments of the present disclosure include imaging devices for imaging within the lumen of a vessel, including both medical and non-medical applications. However, some embodiments of the present disclosure are particularly suited for use in the context of human vasculature. Imaging of the intravascular space, particularly the interior walls of human vasculature can be accomplished by a number of different techniques, including ultrasound (often referred to as intravascular ultrasound ("IVUS") and intracardiac echocardiography ("ICE")) and optical coherence tomography ("OCT"). In other instances, infrared, thermal, or other imaging modalities are utilized.

The electronic, optical, and/or electro-optical components of the present disclosure are often disposed within a distal portion of the flexible elongate member. As used herein, "distal portion" of the flexible elongate member includes any portion of the flexible elongate member from the mid-point to the distal tip. As flexible elongate members can be solid, some embodiments of the present disclosure will include a housing portion at the distal portion for receiving the electronic components. Such housing portions can be tubular structures attached to the distal portion of the elongate member. Some flexible elongate members are tubular and have one or more lumens in which the electronic components can be positioned within the distal portion.

The electronic, optical, and/or electro-optical components and the associated communication lines are sized and shaped to allow for the diameter of the flexible elongate member to be very small. For example, the outside diameter of the elongate member, such as a guidewire or catheter, containing one or more electronic, optical, and/or electro-optical components as described herein are between about 0.0007" (0.0178 mm) and about 0.118" (3.0 mm), with some particular embodiments having outer diameters of approximately 0.014" (0.3556 mm) and approximately 0.018" (0.4572 mm)). As such, the flexible elongate members incorporating the electronic, optical, and/or electro-optical component(s) of the present application are suitable for use in a wide variety of lumens within a human patient besides those that are part or immediately surround the heart, including veins and arteries of the extremities, renal arteries, blood vessels in and around the brain, and other lumens.

"Connected" and variations thereof as used herein includes direct connections, such as being glued or otherwise fastened directly to, on, within, etc. another element, as well as indirect connections where one or more elements are disposed between the connected elements.

"Secured" and variations thereof as used herein includes methods by which an element is directly secured to another element, such as being glued or otherwise fastened directly to, on, within, etc. another element, as well as indirect techniques of securing two elements together where one or more elements are disposed between the secured elements.

Reference will now be made to a particular embodiments of the concepts incorporated into an intravascular ultrasound system. However, the illustrated embodiments and uses thereof are provided as examples only. Without limitation on other systems and uses, such as but without limitation, imaging within any vessel, artery, vein, lumen, passage, tissue or organ within the body. While the following embodiments may refer to a blood vessel and a blood vessel wall for illustrative purposes, any other tissue structure may be envisioned to be imaged according to methods disclosed herein. More generally, any volume within a patient's body may be imaged according to embodiments disclosed herein, the volume including vessels, cavities, lumens, and any other tissue structures, as one of ordinary skill may recognize.

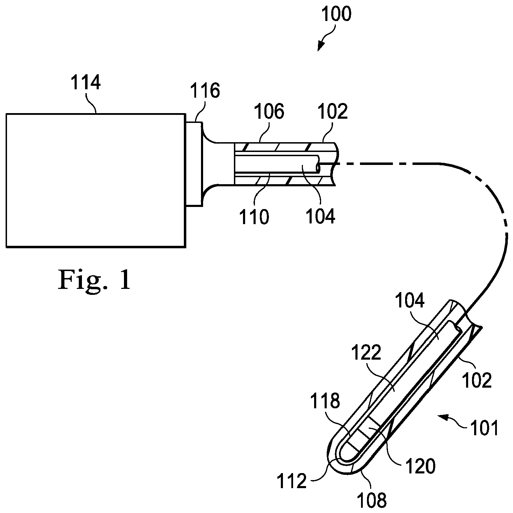

Referring now to Fig. 1, a rotational probe 100 for insertion into a patient for diagnostic imaging is shown. In some embodiments, the rotational probe 100 is an intravascular ultrasound (IVUS) probe. The probe 100 comprises a catheter 101 having a catheter body 102 and an elongate drive shaft or shaft 104. The catheter body 102 is flexible and has both a proximal portion 106 and a distal portion 108. The catheter body 102 is a sheath surrounding the shaft 104. For explanatory purposes, the catheter body 102 in FIG. 1 is illustrated as visually transparent such that the shaft 104 disposed therein can be seen, although it will be appreciated that the catheter body 102 may or may not be visually transparent. The shaft 104 is flushed with a sterile fluid, such as saline, within the catheter body 102. The fluid serves to eliminate the presence of air pockets around the shaft 104 that adversely affect image quality. The fluid can also act as a lubricant. The shaft 104 has a proximal portion 110 disposed within the proximal portion 106 of the catheter body 102 and a distal portion 112 disposed within the distal portion 108 of the catheter body 102.

The distal portion 108 of the catheter body 102 and the distal portion 112 of the shaft 104 are inserted into a patient during the operation of the probe 100. The usable length of the probe 100 (the portion that can be inserted into a patient) can be any suitable length and can be varied depending upon the application. The distal portion 112 of the shaft 104 includes a work element 118.

The proximal portion 106 of the catheter body 102 and the proximal portion 110 of the shaft 104 are connected to an interface module 114 (sometimes referred to as a patient interface module or PIM). The proximal portions 106, 110 are fitted with a catheter hub 116 that is removably connected to the interface module 114. In some embodiments, the interface module 114 couples the probe 100 to a control system and/or a monitor (not shown) for direct user control and image viewing.

The rotation of the shaft 104 within the catheter body 102 is controlled by the interface module 114, which provides a plurality of user interface controls that can be manipulated by a user. The interface module 114 also communicates with the work element 118 by sending to and receiving signals from the work element 118 via conductors within the shaft 104. In some embodiments, the signals to and from the work element 118 are electrical signals and the conductors within the shaft 104 are electrical conductors such as metal wires. In some embodiments, the signals to and from the work element 118 are optical signals and the conductors within the shaft 104 are optical fibers. The interface module 114 can receive, analyze, and/or display information received through the shaft 104. It will be appreciated that any suitable functionality, controls, information processing and analysis, and display can be incorporated into the interface module 114.

The shaft 104 includes a work element 118, a housing 120, and a drive cable 122. The work element 118 is coupled to the housing 120. The housing 120 is attached to the drive cable 122 at the distal portion 112 of the shaft 104. The drive cable 122 is rotated within the catheter body 102 via the interface module 114 and it in turn rotates the housing 120 and the work element 118. The work element 118 can be of any suitable type, including but not limited to one or more transducer technologies such as PMUT or CMUT. The work element 118 can include either a single transducer or an array. In some embodiments, the work element 118 includes sensor components or optical lens, such as those used in an OCT system.

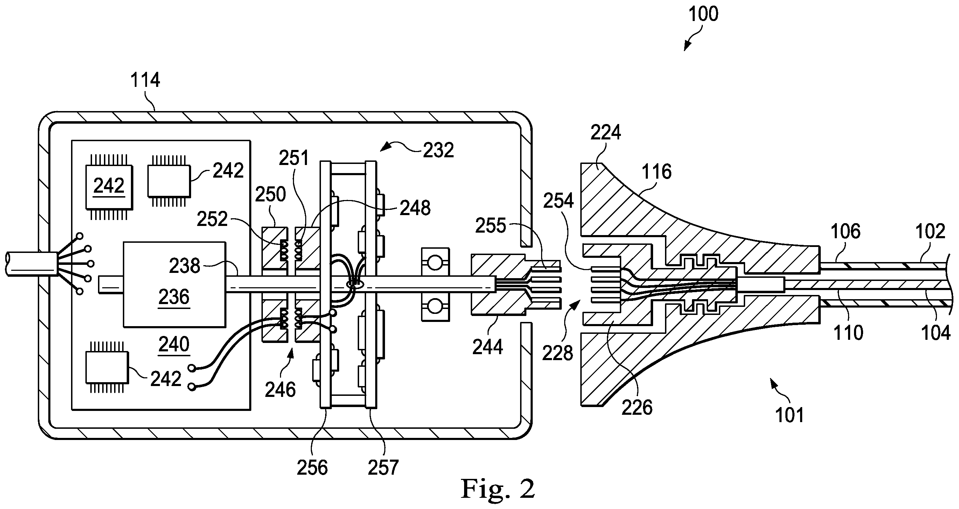

FIG. 2 shows a diagrammatic view of the proximal portion of the probe 100 and the interior of the interface module 114, in accordance with an embodiment. As shown, the catheter hub 116 includes a stationary exterior housing 224, a dog 226, and a connector 228. The connector 228 is represented with four conductive lines, such as 254, shown in this embodiment. It will be appreciated, however, that any suitable number of conductive lines and any type of conductive media can be utilized. For example, an optical coupler, a coaxial cable, or six electrically conductive lines can be utilized in various embodiments.

As shown, the interior of the interface module 114 includes a motor 236, a motor shaft 238, a main printed circuit board (PCB) 240, a spinning element 232, and any other suitable components for the operation of the probe 100. The motor 236 is connected to the motor shaft 238 to rotate the spinning element 232. The main printed circuit board 240 can have any suitable number and type of electronic components 242 including but not limited to the transmitter and the receiver for the work element 118 (FIG. 1).

The spinning element 232 has a complimentary connector 244 for mating with the connector 228 on the catheter hub 116. The connector 244 can have conductive lines, such as 255, that contact the conductive lines, such as 254, on the connector 228. As shown, the spinning element 232 is coupled to a rotary portion 248 of a rotary transformer 246. The rotary portion 248 of the transformer 246 passes signals to and from the stationary portion 250 of the transformer 246 using a set of windings 251 and 252. The stationary portion 250 of the transformer 246 is electrically connected to the printed circuit board 240. It will be appreciated that any suitable number of windings may be used to transmit any suitable number of signals across the transformer 246. Also as shown, the spinning element 232 includes printed circuit boards 256, 257 comprising a plurality of circuit components. It will be appreciated that FIG. 2 is merely an example and is not intended to limit the present disclosure. For example, a pullback mechanism may be employed to pull the shaft 122 proximally within the catheter 102 to generate a longitudinal image of a vessel. More examples of the proximal portion of the probe 100 and the interior of the interface module 114 can be found in U.S. Patent 8,403,856 entitled "Rotational Intravascular Ultrasound Probe with an Active Spinning Element".

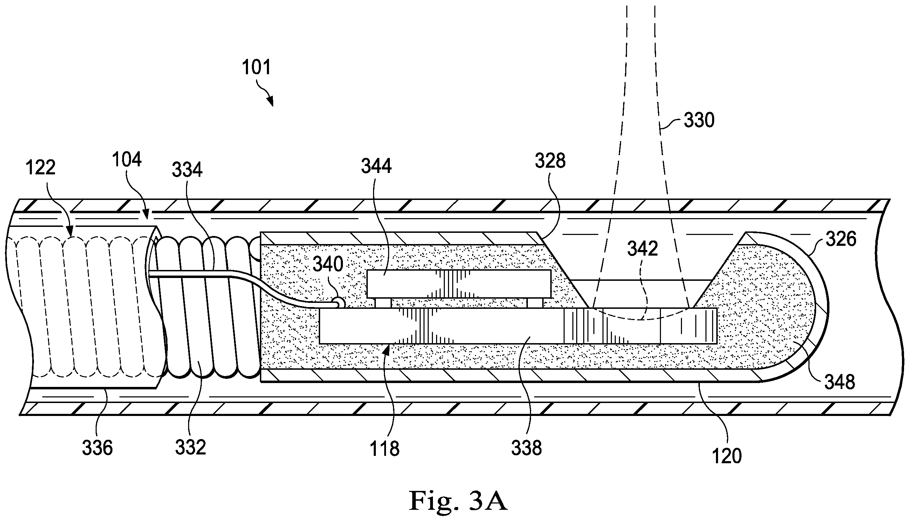

FIG. 3A shows a cross-sectional side view of a distal portion of the catheter 101 according to an embodiment of the present disclosure. In particular, FIG. 3A shows an expanded view of aspects of the distal portion of the shaft 104. In this exemplary embodiment, the shaft 104 is terminated at its distal tip by a housing 120 fabricated from stainless steel and provided with a rounded nose 326 and a cutout 328 for the ultrasound beam 330 to emerge from the housing 120. The drive cable 122 of the shaft 104 includes a torque transmission core 332 and one or more electrical cables 334 secured thereon by a polymer jacket 336. In some embodiments, the electrical cables 334 are secured to the torque transmission core 332 by a plurality of polymer bands instead of a polymer jacket. In some embodiments, the torque transmission core 332 is composed of two or more layers of counter wound stainless steel wires, welded, or otherwise secured to the housing 120 such that rotation of the drive cable 122 also imparts rotation on the housing 120. In the illustrated embodiment, the work element 118 includes a PMUT microelectromechanical system (MEMS) 338 and an application specific integrated circuit (ASIC) 344 mounted thereon. The PMUT MEMS 338 includes a spherically focused transducer 342. The work element 118 is mounted within the housing 120. As shown in FIG. 3A, one of the electrical cables 334 with an optional shield 333 is attached to the work element 118 with a solder 340. The electrical cables 334 extends through an outer portion of the drive cable 122 to the proximal portion of the shaft 104 where it is terminated to the electrical connector 228 (FIG. 2). In the illustrated embodiment, the work element 118 is secured in place relative to the housing 120 by an epoxy 348 or other bonding agent. The epoxy 348 also serves as an acoustic backing material to absorb acoustic reverberations propagating within the housing 120 and as a strain relief for the electrical cable 334 where it is soldered to the work element 118. It will be appreciated that FIG. 3A is merely an example and is not intended to limit the present disclosure. More examples of the distal portion of the shaft 104 and the work element 118 can be found in U.S. Patent Application Publication No. 2013/0303919 entitled "Circuit Architectures and Electrical Interfaces for Rotational Intravascular Ultrasound (IVUS) Devices".

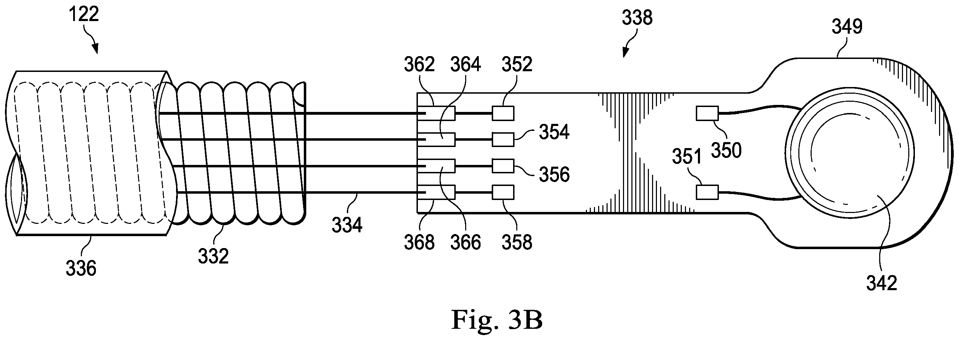

FIG. 3B shows additional aspects of the PMUT MEMS component 338 of the work element 118. The MEMS component 338 in the embodiment of FIG. 3B is a paddle-shaped silicon component with the piezoelectric polymer transducer 342 located in the widened portion 349 of the substrate located at the distal portion of the MEMS component 338. The narrow portion of the substrate positioned proximal of the widened portion 349 is where the ASIC 344 is mounted to the MEMS component 338. In that regard, the MEMS component 338 includes ten bond pads, with bond pads 350, 351, 352, 354, 356, and 358 configured to match up respectively with bond pads on the ASIC 344 for mounting the ASIC 344 thereon, and bond pads 362, 364, 366, and 368 serving as terminations for the four electrical cables 334 of the drive cable 122. In that regard, the four electrical cables 334 of the drive cable 122 are exposed at a distal portion of the drive cable 122, and are soldered or otherwise fixedly attached to bond pads 362, 364, 366, and 368, which are electrically coupled with the bond pads 352, 354, 356, and 358 by conductive traces included on the MEMS substrate. Other embodiments of connecting the electrical cables 334 to the work element 118 are possible, such as those disclosed in U.S. Patent Application Publication No. 2013/0303919 entitled "Circuit Architectures and Electrical Interfaces for Rotational Intravascular Ultrasound (IVUS) Devices."

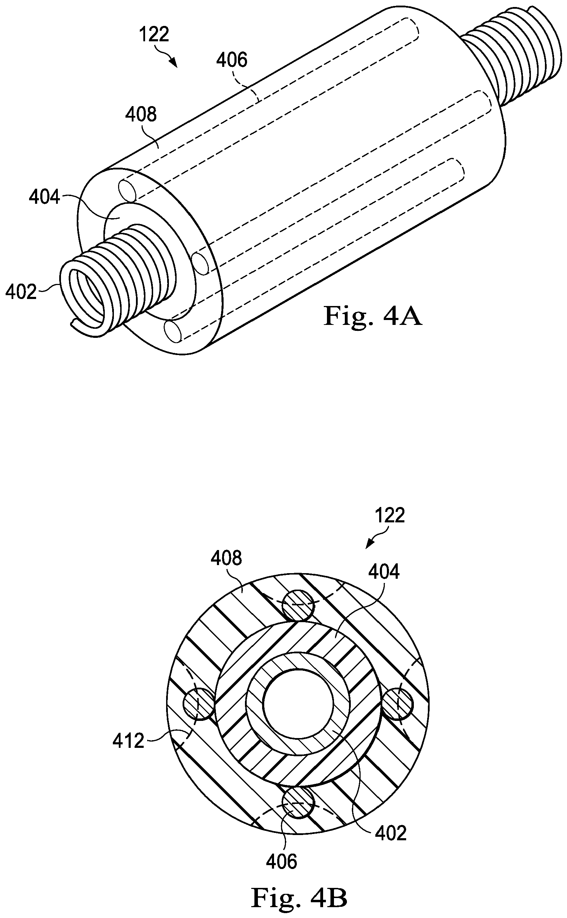

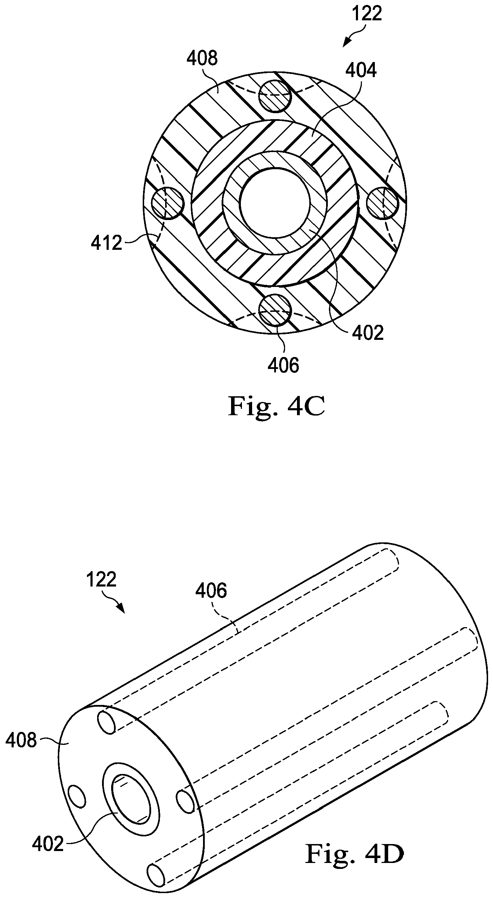

FIG. 4A shows a diagrammatic schematic view of the drive cable 122, according to various aspects of the present disclosure. Referring to FIG. 4A, the drive cable 122 includes a torque transmission core 402, an optional electrical insulating layer 404, one or more conductors 406, and a polymer jacket 408. The torque transmission core 402 possesses a certain torsional stiffness in order to adequately deliver rotational force along the relatively long path traversed by the drive cable 122. At the same time, the torque transmission core 402 is sufficiently flexible to bend around the tight turns presented by the vascular system while maintaining the ability to rotate and to axially translate through the catheter 101 (FIG. 1). The torque transmission core 402 can be made of any suitable material. In an embodiment, the torque transmission core 402 is made of stainless steel, such as two or more layers of counter wound stainless steel wires or braided wires. In an embodiment, the torque transmission core 402 is an optical fiber. The conductors 406 are electrical conductors in some embodiments. In that regard, the conductors 406 may be optionally shielded. In various embodiments, the conductors 406 may be wire (Cu, etc.), carbon nanotube fiber conductors, conductive ink, conductive polymer, conductive film, and/or combinations thereof. In some embodiments, the conductors 406 are optical pathways, such as optical fibers used in OCT systems. In some embodiments, the drive cable 122 includes both electrical conductors 406 and optical conductors 406 in one cable. In some embodiments, the insulating layer 404 serves to electrically isolate the conductors 406 from the torque transmission core 402. The insulating layer 404 may be formed of any suitable material. In some implementations, the insulating layer 404 is a parylene layer. The polymer jacket 408 secures the conductors 406 and the optional electrical insulating layer 404 over the torque transmission core 402. In some embodiments, such as those will be described with reference to FIG. 4C, the polymer jacket 408 can serve as insulating layer for the conductors 406. Furthermore, the polymer jacket 408 also serves to protect the various components of the drive cable 122 from the fluid filled inside the catheter 101. The polymer jacket 408 may be of any polymeric, insulating, and/or dielectric material, such as polyvinyl chloride (PVC), KaptonTM polyimide film from DuPont, ethylene tetrafluoroethylene (ETFE), nylon, or similar polyimide films. In some embodiments, the polymer jacket 408 is an elongate piece, such as a continuous layer in the drive cable 122. In some embodiments, the polymer jacket 408 comprises a plurality of polymer bands that may be separate or be alternatively joined or fused. In yet another embodiment, the polymer jacket 408 is a spiral wrap. In various embodiments, the polymer jacket 408 can be coated, extruded, or shrunk over the torque transmission core 402.

An advantage of the drive cable 122 of FIG. 4A over conventional drive cables is that it is easier to manufacture because the conductors 406 are placed outside the torque transmission core 402, rather than having to be threaded therein as is the case in the conventional drive cables. Furthermore, since there is no need to thread conductors through the torque transmission core 402, the dimensions and tolerance of the drive cable 122 can be reduced, allowing for more space for additional components for the IVUS system. A smaller drive cable 122 also allows for a bigger space between the drive cable and the inside surface of the catheter lumen for easier flushing or injection operations. In addition or alternatively, the drive cable 122 can be made stronger, allowing for more reliable operation and longer usable life.

FIG. 4B shows a cross-sectional view of the drive cable 122 of FIG. 4A, in accordance with an embodiment. Referring to FIG. 4B, in this embodiment, the torque transmission core 402 is shown as a solid core. In alternative embodiments, the torque transmission core 402 is a helical winding having an inner lumen, potentially much smaller than that of existing drive cables. Also shown in FIG. 4B, there are four conductors 406 spaced evenly around the electrical insulating layer 404. In other embodiments, any number of conductors 406 is possible and different arrangement of the conductors 406 is also possible. The polymer jacket 408 wraps around and secures the conductors 406 to the insulating layer 404. In an embodiment, the polymer jacket 408 is a heat shrinkable elongate jacket with a large lumen through which a subassembly of the conductors 406, the insulating layer 404 and the torque transmission core 402 is threaded. The polymer jacket 408 is subsequently heated so as to securely wrap around the subassembly. Also shown in FIG. 4B with dashed lines 412, portions of the polymer jacket 408 are removed at the proximal and/or distal portion of the drive cable 122 to expose the conductors 406. This makes it easier for downstream manufacturing of the rotational probe 100 (FIG. 1), e.g., when the drive cable 122 is to be coupled with the work element 118 (FIG. 3B) or to be terminated with the connector 228 of the catheter hub 116 (FIG. 2).

FIG. 4C shows a cross-sectional view of the drive cable 122 of FIG. 4A, in accordance with another embodiment. Many respects of this embodiment are similar to those of the drive cable 122 of FIG. 4B. However, in this embodiment, the polymer jacket 408 has the conductors 406 embedded therein. The polymer jacket 408 is secured around the insulating layer 404 and the torque transmission core 402 by, e.g., a heat shrink process or any other processes. Having the polymer jacket 408 with the conductors 406 embedded therein further simplifies the manufacturing of the drive cable 122 and the rotational probe 100 (FIG. 1). In this embodiment, the polymer jacket 408 itself may offer sufficient insulation between the torque transmission core 402 and the conductors 406, and therefore, the insulating layer 404 may be unnecessary in some instances.

FIG. 4D shows a diagrammatic schematic view of the drive cable 122, in accordance with an embodiment. Referring to FIG. 4D, in this embodiment, the torque transmission core 402, the conductors 406, and the polymer jacket 408 are formed as one piece. For example, the conductors 406 and the polymer jacket 408 can be co-extruded over the torque transmission core 402 during a manufacturing process.

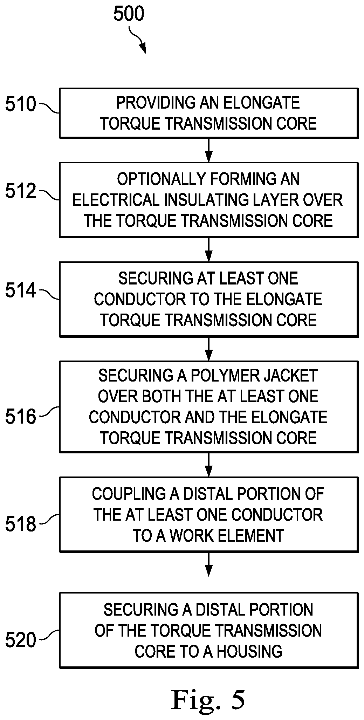

FIG. 5 shows a method 500 of manufacturing a catheter for a rotational probe for insertion into a vessel, such as the catheter 101 (FIG. 1), according to various aspects of the present disclosure. The method 500 is merely an example, and is not intended to limit the present disclosure beyond what is explicitly recited in the claims. Additional operations can be provided before, during, and after the method 500, and some operations described can be replaced, eliminated, or moved around for additional embodiments of the method 500. Various operations of FIG. 5 will be described below in conjunction with FIGS. 1-4D discussed above.

Operation 510 includes providing an elongate torque transmission core, such as the torque transmission core 402 of FIG. 4A. The torque transmission core has desired length and dimension for the catheter to be manufactured. In some embodiments, the torque transmission core is electrically conductive, such as counter wound stainless steel wires. In some embodiments, the torque transmission core is not electrically conductive, such as an optical fiber.

Operation 512 includes optionally forming an electrical insulating layer over the torque transmission core. This is usually the case when the torque transmission core is electrically conductive and the conductors to be assembled onto the torque transmission core are also electrically conductive and are not shielded.

Operation 514 includes securing at least one conductor to the elongate torque transmission core. The number of conductors depends on the intended function of the catheter. For example, an advanced PMUT transducer catheter may need to have four or six conductors. Some catheters may require only one or two conductors. In addition, the conductors are suitable for conducting energy for the intended catheter. In that regard, the conductors may be electrical conductors, waveguides such as optical fibers, or a combination thereof. The at least one conductor may be secured to the torque transmission core by gluing, electrically printing (micro-dispense, aero-jet, ink-jet, transfer, gravure, etc.), or plating a conductive material over the insulating layer, or by helically wrapping the conductor around the torque transmission core.

Operation 516 includes securing a polymer jacket over both the at least one conductor and the elongate torque transmission core. In an embodiment, securing the polymer jacket includes wrapping the polymer jacket over the at least one conductor and the elongate torque transmission core. In an embodiment, securing the polymer jacket includes sliding the polymer jacket over the at least one conductor and the elongate torque transmission core. In an embodiment, securing the polymer jacket further includes heating the polymer jacket so as to axially shrink its dimension. In some embodiments, the polymer jacket has the requisite conductors embedded therein. In such cases, operations 514 and 516 are combined into one operation. In some embodiments, operation 516 secures a plurality of polymer jacket bands over both the at least one conductor and the elongate torque transmission core.

Operation 518 includes coupling a distal portion of the at least one conductor to a work element, such as shown in FIG. 3B. In that regard, a distal portion of the polymer jacket are removed so as to expose the at least one conductor. Subsequently, the conductors are coupled to the work element through appropriate methods, such as soldering.

Operation 520 includes coupling a distal portion of the torque transmission core to a housing that holds the work element, such as shown in FIG. 3A. In some instances, some steps may be performed before operation 520, such as applying epoxy so as to secure the work element and the conductors in the housing. The torque transmission core can be secured to the housing by a suitable method, such as welding.

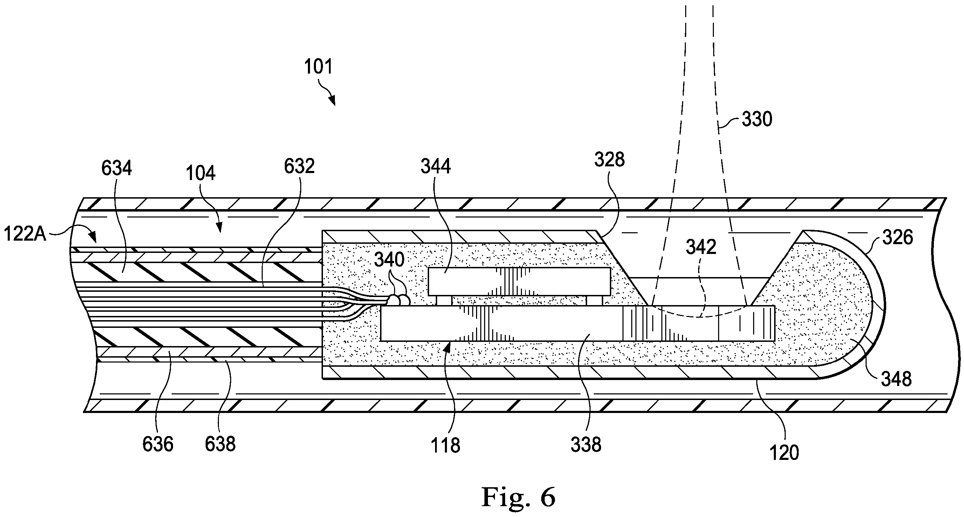

FIG. 6 shows a cross-sectional side view of a distal portion of the catheter 101 according to another embodiment of the present disclosure. Many respects of this embodiment are the same as or similar to those of the embodiment shown in FIG. 3A. Therefore, they are labeled with the same reference numerals for the sake of brevity. However, this embodiment has some distinct features. For example, the drive cable, labeled as 122A and also called data cable in this embodiment, has a different construction than the drive cable 122 in FIG. 3A. Referring to FIG. 6, the drive cable 122A includes one or more conductors 632, a dielectric insulating layer 634, a shield 636, and an outer sheath 638. The conductors 632 are attached to the work element 118 with solders 640 in the distal portion. They also extend through an inner cavity of the drive cable 122A to the proximal portion of the shaft 104 where they are terminated to the electrical connector 228 (FIG. 2). In various embodiments, the drive cable 122A is made strong enough to carry torque needed for the operations of the catheter 101 without a need for a separate torque transmission core thereby achieving a one-piece design with both data transmission and torque transmission capabilities.

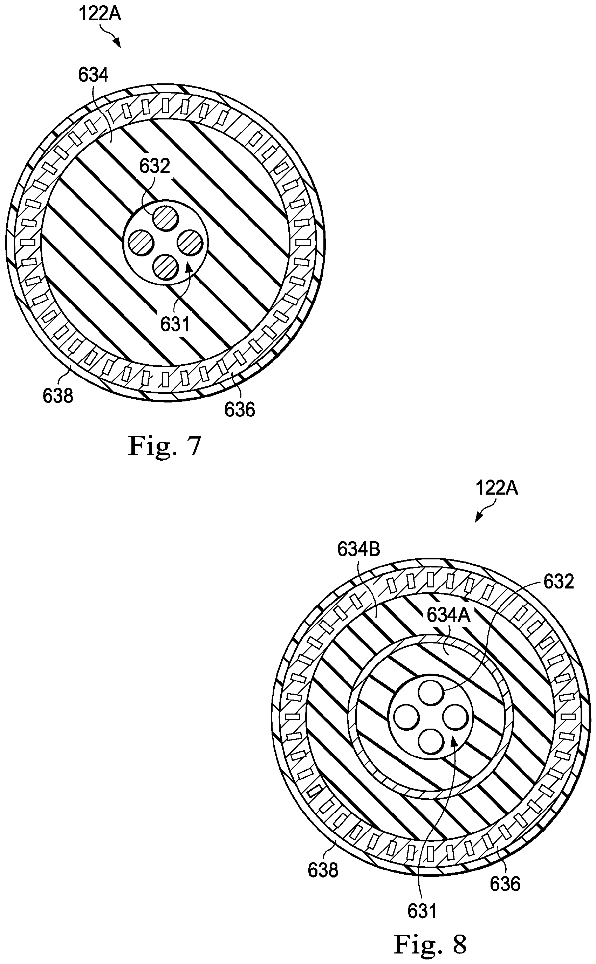

FIG. 7 shows a diagrammatic cross-sectional view of an embodiment of the drive cable 122A. Referring to FIG. 7, shown therein are four conductors 632 in a cavity 631 inside the dielectric insulating layer 634. Each of the conductors 632 may be individually shielded. In an embodiment, the conductors 632 are similar to the inner conductors found in coaxial cables. In an embodiment, the conductors 632 are made of copper, solid or stranded. Although FIG. 7 shows four conductors 632 in the drive cable 122A, this is not intended to be limiting. In various embodiments, a different number of conductors are possible depending on the application. For example, there may be two conductors or six conductors. In an embodiment, there are at least two conductors 632. The conductors 632 may be threaded through the cavity 631. Alternatively, the dielectric insulating layer 634 may be extruded over the conductors 632. The dielectric insulating layer 634 may be made of various materials, such as fluorinated ethylene propylene (FEP), poly tetrafluoroethylene (PTFE), or materials similar to those found in coaxial cables' dielectric layer. In the present embodiment, the dielectric insulating layer 634 is made strong enough to transmit torque, for example, by having a relatively large dimension. In the illustrated embodiment, the insulating layer 634 is also a torque transmission layer that substantially files the volume within shield 636 and has a cross-sectional area greater than the cross-sectional area of the conductors 632. The dielectric insulating layer 634 is reinforced by the shield 636 and the outer sheath 638. The shield 636 may be braided or woven, and may be made of copper, aluminum, or other materials. In an embodiment, the shield 636 is grounded in the proximal portion and serves as an electrical shield for the conductors 632. The outer sheath 638 may be made of PVC, tetrafluoroethylene (TFE), FEP, or a material similar to that of the polymer jacket 408 discussed above. In various embodiments, one or more of the dielectric insulating layer 634, the shield 636, and the outer sheath 638 are made strong enough for transmitting torque. Accordingly, various embodiments of the drive cable 122A provide a one-piece design for both data signal transmission and torque transmission, eliminating the need for a separate torque transmission core.

FIG. 8 shows a diagrammatic cross-sectional view of another embodiment of the drive cable 122A. Referring to FIG. 8, this embodiment includes a strengthening layer 633 embedded in the dielectric insulating layer 634 (or 634A/634B). In an embodiment, the dielectric insulating layer 634 includes two insulating layers 634A and 634B, and the strengthening layer 633 is woven or braided over the insulating layer 634A and is then covered by the insulating layer 634B. In an embodiment, the strengthening layer 633 is made of a conductive material, such as copper, aluminum, or the like. To further this embodiment, the strengthening layer 633 can be made an electrical shield by grounding it in the proximal portion. Non-conductive materials can also be used for the strengthening layer 633, for example, when the shield 636 provides sufficient electrical shield for the conductors 632. Similar to the embodiment shown in FIG. 7, the drive cable 122A in FIG. 8 also provides a one-piece design for both data signal transmission and torque transmission, eliminating the need for a separate torque transmission core.

The foregoing outlines features of several embodiments so that those of ordinary skill in the art may better understand the aspects of the present disclosure. Persons having ordinary skill in the art will also recognize that the apparatus, systems, and methods described above can be modified in various ways. Accordingly, persons having ordinary skill in the art will appreciate that the embodiments encompassed by the present disclosure are not limited to the particular exemplary embodiments described above. In that regard, although illustrative embodiments have been shown and described, a wide range of modification, change, and substitution is contemplated in the foregoing disclosure. It is understood that such variations may be made to the foregoing without departing from the scope of the present disclosure. Accordingly, it is appropriate that the appended claims be construed broadly and in a manner consistent with the present disclosure.

## Claims

1. An elongate catheter, comprising:
a flexible body (102);
a proximal connector (228) adjacent a proximal portion (106) of the flexible body (102); and
an elongate shaft (104) disposed within the flexible body (102), the elongate shaft (104) having a drive cable (122) and a work element (118) coupled to the drive cable (122) adjacent a distal portion (108) of the flexible body (102), the drive cable (122) having a torque transmission core (332, 402) and at least one conductor (406) disposed lengthwise outside of the torque transmission core (332, 402), wherein the at least one conductor (406) is secured to the torque transmission core (332, 402) and the at least one conductor (406) couples the work element (118) to a proximal portion (110) of the elongate shaft (104);
**characterized in that** the drive cable (122) further comprises:
a polymer jacket (408) or a plurality of polymer bands, the polymer jacket (408) or the plurality of polymer bands respectively securing the at least one conductor (406) to the torque transmission core (332, 402).

2. The elongate catheter of claim 1, wherein the at least one conductor (406) is an electrical conductor.

3. The elongate catheter of claim 1, wherein the at least one conductor (406) is an optical fiber.

4. The elongate catheter of claim 1, wherein the drive cable (122) further comprises an electrical insulating layer (634) between the at least one conductor (406) and the torque transmission core (332, 402).

5. The elongate catheter of claim 1, wherein the drive cable (122) comprises a polymer jacket (408), and wherein the at least one conductor (406) is embedded in the polymer jacket (408), and wherein the polymer jacket (408) is secured to the torque transmission core (332, 402).

6. The elongate catheter of claim 1, wherein the torque transmission core (332, 402) is made with stainless steel.

7. The elongate catheter of claim 1, wherein the work element (118) is one of: a piezoelectric micro-machined ultrasound transducer (PMUT) and a capacitive micro-machined ultrasound transducer (CMUT).

8. An intravascular system, comprising:
the elongate catheter (101) according to any of the claims 1 to 7; and
an interface module (114) configured to interface with the proximal connector (228) of the elongate catheter (101), the interface module (114) including: a spinning element (232) configured to be fixedly coupled to the proximal portion (110) of the elongate shaft (104); a stationary element (250) positioned adjacent to and spaced from the spinning element (232), wherein the stationary element (250) is configured to pass signals to and receive signals from the work element (118) through the spinning element (232); and a motor (236) coupled to the spinning element (232) for rotating the spinning element (232) and the elongate shaft (104) when the spinning element (232) is fixedly coupled to the proximal portion (110) of the elongate shaft (104).

## Patentansprüche

1. Länglicher Katheter, umfassend:
einen biegsamen Körper (102);
einen proximalen Verbinder (228) neben einem proximalen Abschnitt (106) des biegsamen Körpers (102); und
einen länglichen Schaft (104), der innerhalb des biegsamen Körpers (102) angeordnet ist, wobei der längliche Schaft (104) ein Antriebskabel (122) und ein Arbeitselement (118) aufweist, das mit dem Antriebskabel (122) neben einem distalen Abschnitt (108) des biegsamen Körpers (102) gekoppelt ist, wobei das Antriebskabel (122) einen Drehmomentübertragungskern (332, 402) und mindestens einen Leiter (406) aufweist, der in Längsrichtung außerhalb des Drehmomentübertragungskerns (332, 402) angeordnet ist, wobei der mindestens eine Leiter (406) an dem Drehmomentübertragungskern (332, 402) befestigt ist und der mindestens eine Leiter (406) das Arbeitselement (118) mit einem proximalen Abschnitt (110) des länglichen Schafts (104) verbindet;
**dadurch gekennzeichnet, dass** das Antriebskabel (122) ferner Folgendes umfasst:
einen Polymermantel (408) oder eine Mehrzahl von Polymerbändern, wobei der Polymermantel (408) oder die Mehrzahl von Polymerbändern jeweils den mindestens einen Leiter (406) an dem Drehmomentübertragungskern (332, 402) befestigt.

2. Länglicher Katheter nach Anspruch 1, wobei der mindestens eine Leiter (406) ein elektrischer Leiter ist.

3. Länglicher Katheter nach Anspruch 1, wobei der mindestens eine Leiter (406) eine Glasfaser ist.

4. Länglicher Katheter nach Anspruch 1, wobei das Antriebskabel (122) ferner eine elektrische Isolierschicht (634) zwischen dem mindestens einen Leiter (406) und dem Drehmomentübertragungskern (332, 402) umfasst.

5. Länglicher Katheter nach Anspruch 1, wobei das Antriebskabel (122) einen Polymermantel (408) umfasst, und wobei der mindestens eine Leiter (406) in den Polymermantel (408) eingebettet ist, und wobei der Polymermantel (408) an dem Drehmomentübertragungskern (332, 402) befestigt ist.

6. Länglicher Katheter nach Anspruch 1, wobei der Drehmomentübertragungskern (332, 402) aus Edelstahl besteht.

7. Länglicher Katheter nach Anspruch 1, wobei das Arbeitselement (118) eines der Folgenden ist: ein piezoelektrischer mikrobearbeiteter Ultraschallwandler (PMUT) und ein kapazitiver mikrobearbeiteter Ultraschallwandler (CMUT).

8. Intravaskuläres System, umfassend:
einen länglichen Katheter (101) nach einem der Ansprüche 1 bis 7; und
ein Schnittstellenmodul (114), das konfiguriert ist, mit dem proximalen Verbinder (228) des länglichen Katheters (101) eine Schnittstelle zu bilden, wobei das Schnittstellenmodul (114) Folgendes enthält: ein Spinnelement (232), das zur festen Kopplung mit dem proximalen Abschnitt (110) des länglichen Schafts (104) konfiguriert ist; ein stationäres Element (250), das an das Spinnelement (232) angrenzend angeordnet und von diesem beabstandet ist, wobei das stationäre Element (250) konfiguriert ist, Signale zu dem Arbeitselement (118) durch das Spinnelement (232) zu leiten und Signale von diesem zu empfangen; und einen mit dem Spinnelement (232) gekoppelten Motor (236) zum Drehen des Spinnelements (232) und des länglichen Schafts (104), wenn das Spinnelement (232) fest mit dem proximalen Abschnitt (110) des länglichen Schafts (104) gekoppelt ist.

## Revendications

1. Cathéter allongé, comprenant :
un corps souple (102) ;
un connecteur proximal (228) adjacent à une partie proximale (106) du corps souple (102) ; et
une tige allongée (104) disposée à l'intérieur du corps souple (102), la tige allongée (104) ayant un câble d'entraînement (122) et un élément de travail (118) couplé au câble d'entraînement (122) adjacent à une partie distale (108) du corps souple (102), le câble d'entraînement (122) ayant un noyau de transmission de couple (332, 402) et au moins un conducteur (406) disposé dans le sens de la longueur à l'extérieur du noyau de transmission de couple (332, 402), dans lequel l'au moins un conducteur (406) est fixé au noyau de transmission de couple (332, 402) et l'au moins un conducteur (406) couple l'élément de travail (118) à une partie proximale (110) de la tige allongée (104) ;
**caractérisé en ce que** le câble d'entraînement (122) comprend en outre :
une gaine de polymère (408) ou une pluralité de bandes de polymère, la gaine de polymère (408) ou la pluralité de bandes de polymère fixant respectivement l'au moins un conducteur (406) au noyau de transmission de couple (332, 402).

2. Cathéter allongé selon la revendication 1, dans lequel l'au moins un conducteur (406) est un conducteur électrique.

3. Cathéter allongé selon la revendication 1, dans lequel l'au moins un conducteur (406) est une fibre optique.

4. Cathéter allongé selon la revendication 1, dans lequel le câble d'entraînement (122) comprend en outre une couche d'isolation électrique (634) entre l'au moins un conducteur (406) et le noyau de transmission de couple (332, 402).

5. Cathéter allongé selon la revendication 1, dans lequel le câble d'entraînement (122) comprend une gaine de polymère (408), et dans lequel l'au moins un conducteur (406) est logé dans la gaine de polymère (408), et dans lequel la gaine de polymère (408) est fixée au noyau de transmission de couple (332, 402).

6. Cathéter allongé selon la revendication 1, dans lequel le noyau de transmission de couple (332, 402) est constitué d'acier inoxydable.

7. Cathéter allongé selon la revendication 1, dans lequel l'élément de travail (118) est l'un d'entre: un transducteur ultrasonore micro-usiné piézoélectrique (PMUT) et un transducteur ultrasonore micro-usiné capacitif (CMUT).

8. Système intravasculaire, comprenant :
le cathéter allongé (101) selon l'une quelconque des revendications 1 à 7 ; et
un module d'interface (114) configuré pour assurer l'interface avec le connecteur proximal (228) du cathéter allongé (101), le module d'interface (114) incluant ; un élément rotatif (232) configuré pour être couplé fixement à la partie proximale (110) de la tige allongée (104) ; un élément stationnaire (250) positionné de manière adjacente à l'élément rotatif (232) et espacé de celui-ci, dans lequel l'élément stationnaire (250) est configuré pour transmettre des signaux à l'élément de travail (118) et recevoir des signaux de celui-ci par le biais de l'élément rotatif (232) ; et un moteur (236) couplé à l'élément rotatif (232) pour faire tourner l'élément rotatif (232) et la tige allongée (104) lorsque l'élément rotatif (232) est couplé fixement à la partie proximale (110) de la tige allongée (104).
